(19) Europäisches Patentamt
European Patent Office
Office européen des brevets

(11) Publication number: 0 043 631 B1

# (12) EUROPEAN PATENT SPECIFICATION

(45) Date of publication of patent specification: 28.09.88

(51) Int. Cl.[4]: C 07 D 309/30

(21) Application number: 81200775.5

(22) Date of filing: 07.07.81

## (54) Process for the preparation of a 6-alkyl-valerolactone.

(30) Priority: 08.07.80 NL 8003929

(43) Date of publication of application:
13.01.82 Bulletin 82/02

(45) Publication of the grant of the patent:
28.09.88 Bulletin 88/39

(84) Designated Contracting States:
BE CH DE FR GB IT LI NL

(56) References cited:
US-A-2 786 852
US-A-3 278 557
CHEMICAL ABSTRACTS, vol. 66, no. 19, May 8, 1967 page 7996/7997, abstract 85509h Columbus, Ohio, USA A.L. VOITSEKHOVSKAYA et al. "Substituted lactones and their transformations. IX. Reactions of Delta-methyl-Delta-valerolactone with polyphosphoric acid"

The file contains technical information submitted after the application was filed and not included in this specification

(73) Proprietor: STAMICARBON B.V.
Mijnweg 1
NL-6167 AC Geleen (NL)

(72) Inventor: Van De Moesdijk, Cornelis G. M.
Drossaert Saldenstraat 7
NL-6181 ER Elsloo (NL)
Inventor: Janssen, Petrus Hubert Joseph
Mauritspark 32
NL-6163 HN Geleen (NL)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 043 631 B1

**Description**

The invention relates to a process for the preparation of a 6-alkyl-valerolactone.

A known process for the preparation of such δ-lactones is described in Japanese Patent Application 49/36678 and comprises reduction of unsaturated keto acids with, for instance, a Raney Ni-Al catalyst in a strongly basic environment, followed by acidification of the reaction mixture obtained with strong acid, for instance sulphuric acid. A large quantity of strong base is required with this known process, which means that after acidification with the strong acid a large quantity of salt of very little value is obtained as byproduct. In addition, the reduction requires a long reaction time, for example 5 hours, and a large quantity of catalyst, for example 90 g per 21 g ketone acid.

According to another known process (see US—A—3,278,557), a δ-oxocarboxylic acid ester can be subjected to catalytic hydrogenation in the liquid phase to form the corresponding δ-lactone. This liquid phase process has the drawback that very long reaction times are required, that elevated pressure and a solvent must be applied and that an expensive step such as filtration is required to upgrade the reaction mixture. In US—A—3,278,557 no yields for these reactions are given. According to experiments by applicant, however, the yields of desired product are very low, while the reaction stopped at 35% conversion.

In US—A—2,786,852 a gas phase cyclization process of laevulinic acid to the corresponding γ-lactone in the presence of a copper containing catalyst is described. This reaction however appears to be only applicable for the preparation of γ-lactones from the corresponding γ-oxocarboxylic acids.

The invention now provides a process for preparation of a 6-alkyl-valerolactone without the above objections.

The process according to the invention for the preparation of a 6-alkyl-valerolactone by catalytic hydrogenation of an δ-oxocarboxylic acid ester is characterized in that a ketone-ester with general formula

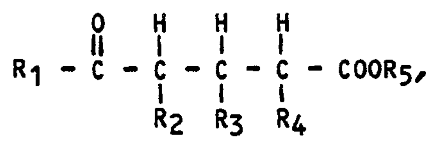

wherein $R_1$ represents an alkyl group with 1—4 carbon atoms, $R_2$, $R_3$ and $R_4$ represent, independently of one another, hydrogen or an alkyl group with 1—4 carbon atoms and $R_5$ represents a hydrocarbon group with at most 8 carbon atoms, is reacted in the gas phase with hydrogen at a temperature of 170—250°C and in the presence of a hydrogenation catalyst containing nickel and/or cobalt and, from the reaction mixture obtained, a 6-alkylvalerolactone is recovered with the general formula

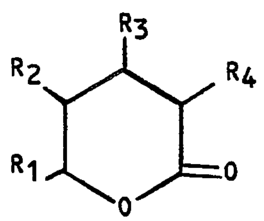

wherein $R_1$, $R_2$, $R_3$ and $R_4$ have the meanings given above.

In the process according to the invention, an alcohol is obtained as byproduct with the general formula $R_5OH$, where $R_5$ has the meaning given above. The alcohol formed can be separated from the reaction mixture by fractional distillation and, if so required, used for the preparation of the ketone ester to be converted according to the invention, for instance by esterifying an alpha-beta unsaturated C-3 carboxylic acid with the alcohol and converting the ester obtained with a ketone as described in British Patent Specification 1,389,510.

In principle, the corresponding keto-acid could be used as starting material instead of the keto-ester (see Chemical Abstracts, Volume 66, No. 19, pp. 7996—7997, Abstract No. 85509 h). However, the conversion and selectivity are here significantly lower than in the process according to the invention. Alkyl groups with at most 4 carbon atoms are preferably used for the hydrocarbon group $R_5$ of the ester to be converted. Other hydrocarbon groups with at most 8 carbon atoms, for example cyclohexyl and benzyl groups, can also be used, but this yields no advantage.

The reaction according to the invention can be performed with known hydrogenation catalysts, for instance catalysts containing a metal or a compound of a metal from Group VIII or the first auxiliary group of the periodic table of elements according to Mendeleev. Nickel and/or cobalt containing catalysts are very suitable. The catalysts can be applied on a support material. Examples of suitable support materials are activated carbon, graphite, silicon oxide, aluminium oxide, magnesium oxide and mixtures of these materials.

The process according to the invention can be carried out at various temperatures in the range 150—300°C. The temperature is preferably chosen in the range 170—250°C because a high conversion and a good yield can then be achieved.

For practical realisation of the reaction according to the invention, the methods known per se for gasphase reactions can be considered, for example the method in which the starting product is passed in a gaseous state, if necessary diluted with an inert gas such as nitrogen, together with the hydrogen over the catalyst in the form of a fixed bed. The space velocity can here be varied between, for instance, 0.01 and 2 gram ketone ester per millilitre catalyst material (compacted volume) per hour. The quantity of hydrogen can also be varied, for example between 1 and 15 mole hydrogen per mole ketone ester to be converted.

By cooling the gaseous reaction mixture, a condensate can be obtained, and hydrogen-containing gas that can be recycled. From the condensate, the desired product can be recovered, for example by fractional distillation. It is also possible to recover the desired product from the condensate by extraction.

The compounds obtained according to the invention can be used for various purposes, for example in the flavour and fragrance industry.

The invention is further elucidated in the following examples.

Comparative Example A

A gaseous mixture of hydrogen and methyl ester of 4-oxopentane-1-carboxylic acid is passed for 95 hours from top to bottom through a vertical tubular reactor (18 mm diameter, 400 mm length) in which there is a zone of 25 ml (compacted volume) catalyst. The catalyst zone is bounded on both sides by a zone of 25 ml inert ceramic material. The catalyst applied is platinum on a support of medium-porous silica (activated by passage of hydrogen for 16 hours at 350°C, 1 wt.% Pt) in the form of particles with a diameter of 3—8 mm.

The gaseous mixture (6 mole hydrogen per mole methyl ester) is obtained by evaporating liquid methyl ester and mixing the vapour with hydrogen. Per ml (compacted volume) catalyst, 0.2 gram methyl ester is passed through per hour. During the first 70 hours, the temperature of the catalyst is kept, by means of a heating jacket round the reactor, at 200°C and thereafter at 250°C.

The composition of the reaction mixture obtained is determined several times by passing the reaction mixture obtained for 2 hours, under constant reaction conditions, through two series-arranged vessels cooled respectively to 0°C and −80°C, and analysing the condensed product thus obtained gas-chromatographically.

From this analysis, the weight of the quantity of ester passed over in the 2-hour period and the weight of the condensed product recovered in this period, the conversion of the ester and the lactone yield can be calculated.

Conversion is understood to mean the quantity of ester converted (quantity of ester passed over minus quantity of ester in the condensed product), expressed as a percentage of the quantity of ester passed over. The lactone yield is understood to mean the quantity of lactone in the condensed product expressed as a percentage of the quantity of lactone that can theoretically be formed from the quantity of ester converted.

The table below shows the conversion and yield data, as well as the operating time prior to the gaseous reaction mixture being passed through the cooled vessels for the 2-hour period.

Table

| operating time in hours | conversion in % | lactone yield in % |
|---|---|---|
| 17.5 | 10.9 | 90 |
| 41.5 | 8.7 | 91 |
| 68.0 | 7.8 | 90 |
| 89.5 | 20.4 | 47.2 |

Example I

In the manner described in Comparative Example A, the methyl ester of 4-oxo-pentane-1-carboxylic acid is passed for 155 hours over a nickel-on-silica catalyst (10 wt.% Ni, Houdry type H 1170). The reaction conditions are modified as indicated in the following table 10—20 hours prior to the reaction mixture being passed through the cooled vessels. This table also shows the conversion and lactone yield data.

Table

| operating time in hours | temperature in °C | space velocity in gram ester per ml cat per hour | mole hydrogen per mole ester | conversion in % | lactone yield in % |
|---|---|---|---|---|---|
| 16 | 180 | 0.2 | 6 | 77.5 | 94.4 |
| 40 | 180 | 0.4 | 3 | 62.8 | 94.3 |
| 61.5 | 180 | 0.4 | 3 | 63.4 | 90.0 |
| 80 | 200 | 0.4 | 3 | 68.7 | 93.4 |
| 104 | 200 | 0.2 | 6 | 89.9 | 87.8 |
| 128.5 | 200 | 0.2 | 6 | 75.3 | 84.5 |
| 153 | 180 | 0.2 | 6 | 77.8 | 97.8 |

Example II

Example I is repeated for 650 hours at 200°C, with a space velocity of 0.2 gram ester per ml catalyst per hour and with application of 6 mole hydrogen per mole ester. The results are given in the table below.

Table

| operating time in hours | conversion in % | lactone yield in % |
|---|---|---|
| 88 | 87.6 | 95.3 |
| 183 | 89.2 | 98.5 |
| 303 | 87.9 | 93.1 |
| 423 | 88.9 | 96.4 |
| 519 | 87.7 | 98.5 |
| 640 | 87.9 | 97.4 |

Example III

In the manner described in Comparative Example A, the isopropyl ester of 4-oxopentane-1-carboxylic acid is passed over a nickel-on-silica catalyst (Houdry type H 1170, 10 wt.% Ni) with a space velocity of 0.2 gram ester per ml catalyst per hour. Per mole ester, 6 mole hydrogen is applied. The temperature is chosen variously and kept constant 10—20 hours prior to the reaction mixture being passed through the cooled vessels. Further data and the results are given in the table below.

Table

| operating time in hours | temperature in °C | conversion in % | lactone yield in % |
|---|---|---|---|
| 19 | 200 | 77.8 | 60.5 |
| 40 | 220 | 81.9 | 52.9 |
| 68.5 | 180 | 63.1 | 79.8 |

Example IV

In the manner described in Comparative Example A, the methyl ester of 4-oxopentane-1-carboxylic acid is passed for 45-hours over a cobalt-on-alumina catalyst (10 wt.% Co on sulphur-free $\gamma$-$Al_{2}O_{3}$ with an internal surface area of 160 $m^2$ per gram) in the form of tablets (diameter 3 mm, thickness 2 mm). Per mole ester, 6 mole hydrogen is applied. The space velocity is kept at 0.18 gram ester per ml catalyst per hour. The temperature is chosen variously and kept constant about 15 hours prior to the reaction mixture being passed through the cooled vessels. Further data and the results are given in the table below.

Table

| operating time in hours | temperature in °C | conversion in % | lactone yield in % |
|---|---|---|---|
| 18 | 200 | 74.7 | 89.1 |
| 43 | 230 | 75.1 | 78.0 |

Example V

Example IV is repeated at various temperatures with a cobalt-on-medium-porous-silica catalyst (10 wt.% Co) in the form of granules of 3—8 mm and applying a space velocity of 0.14 gram ester per ml catalyst per hour. The results and further data are given in the table below.

Table

| operating time in hours | temperature in °C | conversion in % | lactone yield in % |
|---|---|---|---|
| 20.5 | 210 | 92.7 | 94.1 |
| 43 | 230 | 90.5 | 94.1 |

Comparative Example B

In the manner of Comparative Example A, a mixture of hydrogen and the methyl ester of 4-oxopentane-1-carboxylic acid is passed for 20 hours over a copper-on-magnesium-oxide catalyst (38 wt.% Cu) in the form of tablets (diameter 5 mm, thickness 3 mm). Per mole ester, 6 mole hydrogen is applied. The temperature is kept at 200°C while 0.2 gram ester per ml catalyst per hour is passed over. After 18 hours' operating time, the conversion and lactone yield are determined as described in Comparative Example A. These are respectively 29.3% and 58.6%.

**Claims**

1. Process for the preparation of a 6-alkylvalerolactone by catalytic hydrogenation of an δ-oxocarboxylic acid ester, characterized in that a ketone-ester with general formula

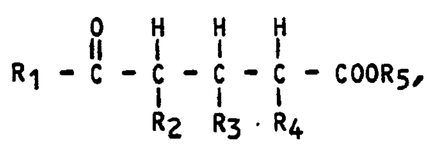

wherein $R_1$ represents an alkyl group with 1—4 carbon atoms, $R_2$, $R_3$ and $R_4$ represent, independently of one another, hydrogen or an alkyl group with 1—4 carbon atoms and $R_5$ represents a hydrocarbon group with at most 8 carbon atoms, is reacted in the gas phase with hydrogen at a temperature of 170—250°C and in the presence of a hydrogenation catalyst containing nickel and/or cobalt and, from the reaction mixture obtained, a 6-alkylvalerolactone is recovered with the general formula

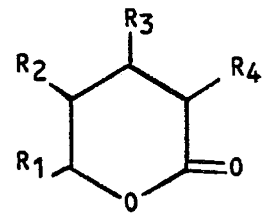

wherein $R_1$, $R_2$, $R_3$ and $R_4$ have the meanings given above.

2. Process according to claim 1, characterized in that the catalyst is applied in the form of a fixed bed and a space velocity of 0.01—2 gram ketone-ester per ml catalyst material per hour is applied.

3. Process according to any of claims 1—2, characterized in that the starting material used is a ketone-ester in which the group $R_5$ represents an alkyl group with at most 4 carbon atoms.

**Revendications**

1. Procédé de préparation d'une 6-alkyl-valérolactone par hydrogénation catalytique d'un acide delta-oxocarboxylique, caractérisé en ce qu'on fait réagir en phase gazeuse un cétone-ester de formule générale:

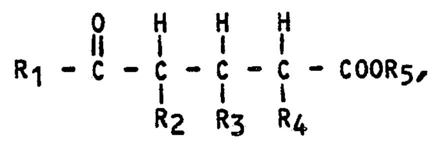

dans laquelle $R_1$ représente un radical alkyle de 1 à 4 atomes de carbone, $R_2$, $R_3$ et $R_4$ représentent chacun indépendamment les uns des autres un atome d'hydrogène ou un radical alkyle contenant de 1 à 4 atomes de carbone et $R_5$ est un radical hydrocarboné contenant au plus 8 atomes de carbone avec de l'hydrogène à une température de 170 à 250°C et en présence d'un catalyseur d'hydrogénation contenant du nickel et/ou du cobalt, et à partir du mélange de réaction ainsi obtenu, on récupère une 6-alkyl-valérolactone de formule générale:

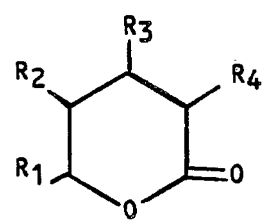

dans laquelle $R_1$, $R_2$, $R_3$ et $R_4$ ont les mêmes significations que ci-dessus.

2. Procédé selon la revendication 1, caractérisé en ce qu'on applique le catalyseur sous forme d'un lit fixe et à une vitesse spatiale de 0,01 à 2 g de cétone-ester par ml de catalyseur à l'heure.

3. Procédé selon la revendication 1 ou 2, caractérisé en ce que la matière de départ utilisée est un cétone-ester dont le radical $R_5$ est un radical alkyle contenant au maximum 4 atomes de carbone.

**Patentansprüche**

1. Verfahren zur Herstellung eines 6-Alkylvalerolactons durch katalytische Hydrierung eines δ-Oxocarbonsäureesters, dadurch gekennzeichnet, daß man einen Ketoester der allgemeinen Formel

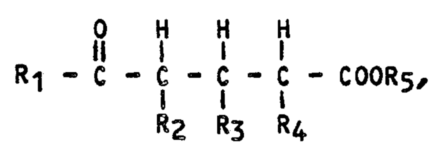

worin $R_1$ eine Alkylgruppe mit 1—4 Kohlenstoffatomen, $R_2$, $R_3$ and $R_4$ unabhängig voneinander für Wasserstoff oder eine Alkylgruppe mit höchstens 1—4 Kohlenstoffatomen stehen und $R_5$ eine Kohlenwasserstoffgruppe mit höchstens 8 Kohlenstoffatomen darstellt, in der Gasphase mit Wasserstoff bei einer Temperatur von 170—250°C und in Gegenwart eines Nickel und/oder Kobalt enthaltenden Hydrierungskatalysators umsetzt und aus dem erhaltenen Reaktionsgemisch ein 6-Alkyl-valerolacton der allgemeinen Formel

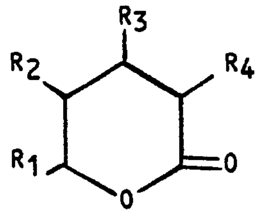

worin $R_1$, $R_2$, $R_3$ and $R_4$ die oben angegebenen Bedeutungen besitzen, gewinnt.

2. Verfahren nach Anspruch 1, dadurch gekennzeichnet, daß der Katalysator in Form eines Festbettes eingesetzt und eine Raumgeschwindigkeit von 0,01—2 Gramm Ketoester pro ml Katalysatormaterial angewendet wird.

3. Verfahren nach einem der Ansprüche 1—2, dadurch gekennzeichnet, daß ein Ketoester als Ausgangsmaterial eingesetzt wird, in welchem die Gruppe $R_5$ eine Alkylgruppe mit höchstens 4 Kohlenstoffatomen darstellt.